# FASCICULE DE BREVET EUROPEEN

(11) **EP 1 250 144 B1**
(45) Date de publication et mention de la délivrance du brevet: **15.12.2004**
(21) Numéro de dépôt: 01907634.8
(22) Date de dépôt: 18.01.2001
(51) Int. Cl.: A61K 35/78, A61K 7/48, A61P 13/08, A61P 17/00

(54) **HUILE VEGETALE POUR LA PREPARATION D'UNE COMPOSITION INHIBITRICE DE LA 5 ALPHA-REDUCTASE**
PFLANZENÖL FÜR DIE HERSTELLUNG EINER HEMMENDER ZUSAMMENSETZUNG DER 5--ALPHA REDUKTASE
VEGETABLE OIL FOR PREPARING A COMPOSITION INHIBITING 5$G(A)- REDUCTASE ACTIVITY

(30) Priorité: 18.01.2000 FR 0000574
(43) Date de publication de la demande: 23.10.2002
(73) Titulaire: Laboratoires Expanscience, 92419 Courbevoie Cedex (FR)
(72) Inventeur: PICCIRILLI, Antoine, F-78000 Versailles (FR); LEGRAND, Jacques, F-61290 Neuilly-sur-Eure (FR); MSIKA, Philippe, F-75018 Paris (FR)
(74) Mandataire: Warcoin, Jacques
(86) Numéro de dépôt international: PCT/FR2001/000169
(87) Numéro de publication internationale: WO 2001/052873

(56) Documents cités:
- DATABASE BIOSIS [en ligne] BIOSCIENCES INFORMATION SERVICE, PHILADELPHIA, PA, US; 1997 SUNITHA T ET AL: "Lipid profile of rats fed blends of rice brain oil in combination with sunflower and safflower oil." Database accession no. PREV199800307524 XP002149678 & PLANT FOODS FOR HUMAN NUTRITION (DORDRECHT), vol. 51, no. 3, 1997, pages 219-230, ISSN: 0921-9668

## Description

La présente invention se rapporte à l'utilisation d'au moins un d'un produit d'huile végétale pour la préparation d'une composition destinée à inhiber l'activité de la 5α-réductase, notamment pour le traitement de l'hypertrophie prostatique, de l'adénome prostatique, de l'acné, de l'hyperséborrhée, de l'alopécie, de l'hirsutisme.

L'invention se rapporte également à des méthodes de traitement cosmétique, notamment de la peau grasse, ainsi qu'à l'utilisation des produits d'huile végétale décrits en tant qu'additifs dans un aliment pour l'être humain et/ou l'animal.

La 5α-réductase est une enzyme microsomiale NADPH dépendante qui existe sous forme de deux isoenzymes synthétisées à partir de deux gènes différents.

L'isoenzyme de type 1 de la 5α-réductase est retrouvée essentiellement dans le foie et la peau, plus particulièrement dans les glandes sébacées de la peau non génitale et du cuir chevelu, et apparaît à la puberté. L'isoenzyme de type 2 est prédominante dans la prostate et au niveau de la peau des territoires sexuels différenciés : région génitale, barbe, et joue un rôle dans la différenciation sexuelle. La répartition des isoenzymes de type 1 et 2 de la 5a-réductase au niveau de la peau et des annexes cutanées chez l'homme peut être illustrée par le tableau ci-après.

Il existe un certain nombre de pathologies pour lesquelles une exagération congénitale ou acquise de l'activité de la 5α-réductase est responsable en totalité ou en majorité des troubles observés.

Par exemple, chez l'homme, cette enzyme 5α-réductase, principalement localisée dans les tissus génitaux et dans la peau, catalyse l'hydroxylation de la testostérone en 5α-réductase dihydrotestostérone (DHT). Or, comme la DHT est un androgène bien plus actif que la testostérone (environ 2 fois plus), les effets de cette dernière sont amplifiés dans les tissus où est produite la DHT. Une activité trop élevée de la 5α-réductase provoque ainsi des teneurs en androgène sous forme de DHT trop élevées dans la prostate, d'où une surstimulation de cette dernière se traduisant en une croissance indésirable pouvant mener à la pathologie de l'hypertrophie prostatique, voire à l'adénome prostatique, nécessitant le plus souvent une intervention chirurgicale.

**Tableau 1 :**

| **répartition des isoenzymes de type 1 et 2 de la 5α-réductase au niveau de la peau et des annexes cutanées chez l'homme** | | | |
|---|---|---|---|
| | | HS-αr1 | H5-αr2 |
| EPIDERME | Couche basale | ++ | + |
| | Couche spineuse | + | ++ |
| | Couche granuleuse | + | - |
| | Couche cornée | - | - |
| DERME | Fibroblastes | ++ | - |
| GLANDES SEBACEES | Cellules basales | ++ | + |
| | Cellules glandulaires | ++ | - |
| GLANDES SUDORALES ECCRINES | Canal excréteur | - | - |
| | Cellules sécrétrices | ++ | - |
| | Cellules myoépithéliales | ++ | + |
| FOLLICULE PILEUX | Papille dermique | + | + ? |
| | Cellules de la matrice | ++ | + |
| | Gaine épithéliale interne | ± | +++ |
| | Gaine épithéliale externe | ++ | - |
| | Muscle arrecteur | + | - |

D'autres pathologies, de type dermatologique, peuvent être observées chez l'homme ou la femme comme résultant d'une suractivité de la 5α-réductase à savoir, en particulier l'acné, l'hirsutisme ou encore l'alopécie.

Dans la peau, l'activité de la 5α-réductase est plus importante dans la glande sébacée que dans les autres structures. Par ailleurs, les glandes séborrhéiques montrent une activé 5α-réductase plus importante que celles des autres territoires cutanés. Par conséquent, le niveau de sécrétion sébacée physiologique semble étroitement lié à l'activité de cette enzyme.

Chez l'acnéïque, il existe une hyperactivité de la 5α-réductase. Plus qu'une augmentation des taux sériques des androgènes, c'est une augmentation des précurseurs en DHT, facteur principal de la fonction sébacée, qui participent à l'acné.

La peau grasse ou (séborrhée), outre son aspect disgracieux, constitue un terrain sur lequel peuvent survenir des complications. Elle atteint les zones où les glandes sébacées sont nombreuses et résulte principalement d'une surstimulation androgénique de la production sébacée par ces glandes spécifiques. L'hyperséborrhée participe à la survenue des lésions d'acné vulgaire.

Dans le cuir chevelu, on retrouve l'isoenzyme de type 1 de la 5α-réductase au niveau des glandes sébacées, ainsi qu'au niveau du follicule pileux. L'isoenzyme de type 2 de la 5α-réductase est localisée majoritairement au niveau de la gaine épithéliale interne, ainsi qu'au niveau de la papille dermique du cheveu. Cependant cette dernière localisation reste à préciser.

L'alopécie androgénique, dont la physiopathogénie est très voisine de celle de l'acné, est la plus fréquente des alopécies et sans doute celle où la demande de thérapeutique est la plus forte. La 5α-réductase semble jouer un rôle primordial dans cette pathologie. En effet, les hommes atteints d'un déficit génétique en isoenzyme de type 2 de la 5α-réductase ne développent pas d'alopécie androgénétique.

Compte tenu de ce qui précède, la recherche s'est orientée vers la mise au point d'inhibiteurs de la 5α-réductase. Certains stéroïdes comme la progestérone ont été testés dans ce sens, mais sa métabolisation rapide la rend inefficace *in vivo.* Pour être actif, l'inhibiteur de 5α-réductase doit être suffisamment stable pour bloquer l'activité de l'enzyme *in vitro.* Le finastéride, inhibiteur compétitif stéroïdien, rempli cette condition, mais il est plus actif sur l'isoenzyme de type 2 que sur l'isoenzyme de type 1 et ces deux isoenzymes n'ont que 50 % d'homologie sur la séquence de leurs acides aminés. C'est donc surtout dans l'hyperplasie bénigne de la prostate que le finastéride a déjà été testé.

Par ailleurs, on connaît également l'extrait de *Serenoa Repens,* comme référence en tant qu'inhibiteur de la 5α-réductase, l'extrait de *Serenoa Repens* présentant l'avantage, par rapport au finastéride, d'une origine naturelle en tant qu'extrait végétal permettant une meilleure comparaison pour des produits testés également d'origine naturelle. *Serenoa Repens,* également connu sous la dénomination *Sabal serrulatum,* est un petit palmier que l'on peut trouver aux Etats-Unis (Floride) en Afrique du Nord et en Espagne.

On a maintenant trouvé de manière tout à fait surprenante et inattendue que l'utilisation de certains composés d'origine végétale permet d'obtenir un effet remarquable d'inhibition de l'activité de la 5α-réductase procurant ainsi notamment une nouvelle réponse pour le traitement des pathologies et/ou désordres dermatologiques évoqués ci-dessus.

La présente invention se rapporte ainsi à l'utilisation d'au moins un produit d'huile végétale choisi dans le groupe constitué par les oléodistillats d'huile végétale, les insaponifiables d'huile végétale, les lipides furaniques d'huile végétale et les mélanges de ces derniers, pour la préparation d'une composition destinée à inhiber l'activité de la 5α-réductase.

En particulier, l'utilisation selon l'invention est caractérisée en ce que la composition est destinée à inhiber l'isoenzyme de type 1 et/ou l'isoenzyme de type 2 de la 5α-réductase.

Parmi les huiles végétales pouvant être utilisées, on peut citer en particulier l'huile de tournesol, de palme, de palmiste, de noix de coco, de pépins de raisins, de moutarde noire, d'ocillette, de beurre de karité, d'amande douce, de soja, d'avocat, de lupin, d'arachide, de coton, de sésame, d'olive, de maïs, de cacao, de ricin, de Ben, de lin, de colza, de rocouyer, de germe de blé, de carthame, de noix, de noisettes et de navette.

Par "oléodistillat d'huile végétale", on entend selon l'invention une huile végétale ayant été soumise à une étape de concentration de sa fraction insaponifiable.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, phytostérols, tocophérols, les pigments caroténoïdes et xanthophiles.

Les huiles végétales dont l'insaponifiable et/ou l'oléodistillat sont riches en tocophérols et/ou en phytostérols sont particulièrement préférées pour l'utilisation selon l'invention. L'homme du métier comprend aisément que le terme "riche" fait référence à des teneurs en tocophérols et en phytostérols respectivement au-dessus des teneurs moyennes respectives obtenues en considérant l'ensemble des huiles végétales connues de l'homme du métier notamment celles citées ci-dessus.

Différentes méthodes peuvent être utilisées pour concentrer la fraction insaponifiable d'une huile végétale : cristallisation par le froid, extraction liquide-liquide, distillation moléculaire.

La distillation moléculaire est particulièrement préférée, étant réalisée de préférence à une température comprise entre environ 180 et environ 280 °C en maintenant une pression comprise entre environ 10⁻³ et environ 10⁻² mmHg et de préférence de l'ordre de 10⁻³ mmHg. La concentration en insaponifiable du distillat peut atteindre 60%.

Cette distillation moléculaire, ainsi que toute autre distillation moléculaire pour la préparation des produits d'huile végétale à utiliser selon l'invention, comme décrit ci-après, est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme du métier. Par exemple, la demande EP- 0 493 144 décrit un distillateur moléculaire de ce type. D'une manière générale, le produit à distiller est étalée en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'insaponifiable, l'avantage étant que l'huile et l'insaponifiable (ces produits étant réputés fragiles) ne sont pas dégradés au cours de l'évaporation.

Les distillateurs moléculaires de type à film raclé, également connus de l'homme du métier, comprennent une chambre de distillation dotée d'un racleur tournant, permettant l'étalement en continu sur la surface d'évaporation (surface chaude) du produit à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile,etc.). La récupération des résidus et des distillats dans des ballons en verre, se fait par écoulement gravitationnel.

Selon un mode de réalisation particulièrement préféré de la présente invention, on utilise un oléodistillat choisi dans le groupe constitué par les oléodistillats d'huile d'avocat, d'huile de soja, d'huile de lupin, d'huile de tournesol et les mélanges de ces derniers.

En particulier, l'oléodistillat de tournesol est obtenu par distillation moléculaire d'une huile de tournesol alimentaire. Les conditions de distillation sont de préférence les suivantes :
- température de 230 à 250 °C;
- pression de 10⁻³ à 10⁻² mmHg;
- taux de distillation d'environ 5 à 10 % massique.

Le taux de distillation peut être défini de la manière suivante : il s'agit du rapport massique ramené à 100 % de la masse du distillat à la somme (masse du distillat + masse du résidu).

Le distillat ainsi obtenu, c'est à dire l'oléodistillat de tournesol, présente une teneur en insaponifiable comprise entre environ 6 et environ 10 % en poids, la partie restante étant composée par les triglycérides de l'huile de tournesol.

L'insaponifiable d'huile végétale pouvant être utilisée selon l'invention est de préférence choisi dans le groupe constitué par l'insaponifiable d'huile d'avocat, l'insaponifiable d'huile de soja et les mélanges de ces derniers.

La comparaison des teneurs en insaponifiables de différentes huiles végétales : soja, coton, noix de coco, olive et avocat montre un taux très important d'insaponifiable de l'huile d'avocat obtenue par extraction suivant divers procédés connus. Typiquement, les teneurs obtenues s'échelonnent de 2 à 7% d'insaponifiable dans l'huile d'avocat contre 0,5% dans l'huile de coco, 1% dans l'huile de soja, 1% dans l'huile d'olive.

La teneur plus importante en insaponifiable dans l'huile d'avocat par rapport aux autres huiles végétales telles que celles mentionnées ci-dessus s'explique en particulier par la présence, dans l'insaponifiable d'huile d'avocat, de constituants que l'on ne retrouve généralement pas dans l'insaponifiable de nombreuses autres huiles végétales tels que des composés furaniques et des alcools gras polyhydroxylés et qui, à eux seuls, représentent plus de 50% de l'insaponifiable. Les produits propres à cet insaponifiable d'avocat peuvent être répartis en deux fractions chimiques appelées "fraction I" et "fraction H". Les composés actifs pour l'utilisation selon l'invention se trouvent présents dans la fraction H et ses précurseurs. La fraction H apparaît en premier lieu sur un chromatographe en phase gazeuse de l'insaponifiable d'huile d'avocat.

Concernant l'insaponifiable d'huile de soja, on peut remarquer que cet insaponifiable est principalement composé de stérols (40 à 65%) et de tocophérols (≥ 10%). Les principaux stérols sont le β-sitostérol (40 à 70% des stérols totaux), le campéstérol (15 à 30% des stérols totaux) et le stigmastérol (10 à 25% des stérols totaux). Les tocophérols sont présents sous la forme d'un mélange de α-tocophérol (5 à 35% des tocophérols totaux), de γ-tocophérol (45 à 70% des tocophérols totaux) et de δ-tocophérol (10 à 43% des tocophérols totaux).

Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR-2 678 632 au nom des Laboratoires Pharmascience. Ce procédé permet d'obtenir un insaponifiable d'avocat riche en fraction H en comparaison aux procédés classiques de préparation d'insaponifiable d'avocat.

Ainsi, l'insaponifiable d'huile d'avocat utilisé selon l'invention peut être obtenu à partir du fruit frais mais, de préférence, l'insaponifiable d'avocat est préparé à partir du fruit préalablement traité thermiquement, avant l'extraction de l'huile et la saponification, comme décrit dans le brevet FR-2 678 632.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80 °C et de préférence comprise entre environ 80 et environ 120 °C.

On peut également citer le procédé de préparation d'insaponifiable d'huile de soja, obtenu à partir d'un oléodistillat (ou "concentrat") d'insaponifiable d'huile de soja. Ledit oléodistillat ou "concentrat" d'insaponifiable est préparé par distillation moléculaire selon un procédé tel que celui décrit pour l'huile de lupin dans la demande de brevet FR-2 762 512, mais adapté à l'huile de soja. Dans ce procédé, l'huile de soja est distillée dans un distillateur moléculaire de type centrifuge ou à film raclé, à une température comprise entre environ 210 et 250° C et sous un vide poussé, compris entre 0,01 et 0,001 millimètres de mercure (soit 0,13 à 1,3 Pa). Le distillat obtenu présente une teneur en insaponifiable comprise entre 5 et 30% en poids et constitue donc un oléodistillat (ou "concentrat") d'insaponifiable d'huile de soja. Ce dernier est ensuite saponifié selon un procédé classique de saponification, en présence de potasse éthanolique. Le mélange obtenu est extrait par le dichloroéthane dans une colonne à contre-courant. La phase solvant est enfin désolvantée par passage dans un évaporateur à film tombant afin de récupérer l'insaponifiable de soja.

Selon un mode de réalisation préféré de la présente invention, l'insaponifiable d'huile végétale est un mélange d'insaponifiables d'huiles d'avocat et de soja, le rapport pondéral d'insaponifiable d'huile d'avocat à l'insaponifiable d'huile de soja étant compris entre environ 0,1 et environ 9, et de préférence compris entre environ 0,25 et environ 0,6.

En particulier, on peut avantageusement utiliser le mélange d'insaponifiables d'huiles d'avocat et de soja tel que commercialisé par la société Laboratoires Pharmascience sous la dénomination "Piascledine 300®" qui consiste en un mélange de 33,3% en poids d'insaponifiable d'avocat et de 66,6% en poids d'insaponifiable de soja, par rapport au poids total du mélange (les 0,1% restants étant constitués de silice colloïdale et de butylhydroxytoluène).

Par "lipides furaniques d'huile végétale", on entend selon l'invention des composés comprenant une chaîne principale linéaire hydrocarbonée en C₁₁ - C₁₉, saturée ou comprenant une ou plusieurs insaturations éthyléniques ou acétyléniques, et un groupe 2-furanyle à l'une de ses extrémités. Parmi les lipides furaniques d'huile végétale pouvant être utilisé selon l'invention, on préfère tout particulièrement les lipide furaniques de l'avocat. L'avocat comprend en effet des lipides particuliers de type furanique, dont le principal composant est un furane linoléique :

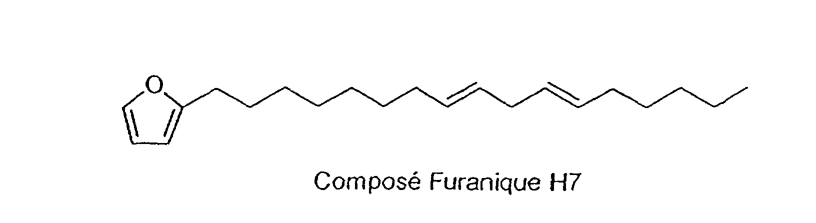

Les dérivés furaniques de l'huile d'avocat ont été décrits notamment dans Farines, M. et al, 1995, J. of Am. Oil Chem. Soc. 72, 473. Il est aujourd'hui bien établi que la présence de ces composés furaniques dans les feuilles ou le fruit dépend non seulement de la variété (les variétés *Hass et Fuerte* étant les plus riches en composés furaniques) mais aussi du mode d'obtention de l'huile ou d'un autre extrait végétal de l'avocat (extrait hexanique ou éthanolique des feuilles d'avocat).

En effet, on sait que ces lipides furaniques sont des métabolites de composés initialement présents dans le fruit et les feuilles qui, sous l'effet de la chaleur, se se déshydratent et se cyclisent en dérivés furaniques.

Par exemple, le furane linoléique est issu de la transformation thermique du précurseur suivant :

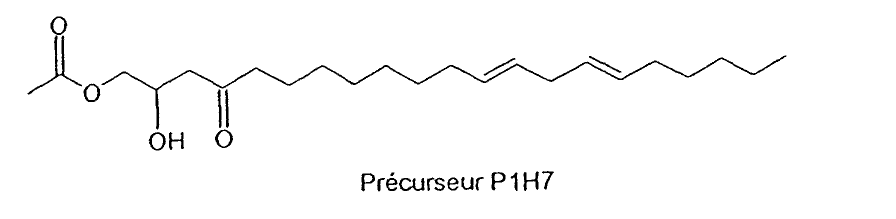

Par "lipides furaniques d'avocat", on entend en particulier selon l'invention les composants répondant à la formule : 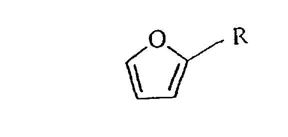 dans laquelle R est une chaîne linéaire hydrocarbonée en C₁₁-C₁₉ de préférence C₁₃-C₁₇ saturée ou comprenant une ou plusieurs insaturations éthyléniques ou acétyléniques.

Les procédés connus pour obtenir ces composés spécifiques à partir du fruit ou de l'huile du fruit de l'avocat se résument soit à la chromatographie préparative, soit à des procédés industriels permettant d'obtenir ces lipides furaniques en mélange avec les autres composés insaponifiables d'avocat, avec une teneur maximale en lipides furaniques comprise au mieux entre 50 et environ 65% en poids seulement.

Un nouveau procédé de préparation de ces lipides furaniques de l'avocat a fait l'objet du dépôt d'une demande de brevet ce même jour. Il consiste pour l'essentiel en un procédé d'extraction sélective des lipides furaniques d'avocat, caractérisé en ce qu'il comprend les étapes consistant à préparer un insaponifiable d'avocat, puis à soumettre l'insaponifiable d'avocat à une étape de distillation moléculaire en utilisant des moyens de température réglés pour une température comprise entre 100 et 160°C et des moyens de pression réglés pour une pression comprise entre 10⁻³ et 510⁻² mmHg.

Cette étape de distillation moléculaire mettant en oeuvre des conditions de températures et de pressions spécifiques, constitue une caractéristique essentielle de ce procédé, en combinaison avec l'étape préalable de préparation de l'insaponifiable déjà décrite ci-dessus.

Selon l'invention, le produit d'huile végétale tel que décrit ci-dessus est utilisé selon une proportion comprise entre environ 0,001 et environ 100 % en poids (possibilité d'utilisation sous forme pure, sans excipient), de préférence entre environ 0,01 et environ 70 % en poids, et plus particulièrement encore entre environ 0,1 et 10 % en poids, par rapport au poids total de la composition.

La composition préparée par l'utilisation selon l'invention peut en outre comprendre un excipient pharmaceutiquement, dermatologiquement ou cosmétiquement acceptable. On peut utiliser tout excipient adapté pour les formes galéniques connues de l'homme de métier, en vue d'une administration par voie topique, orale, entérale ou parentérale, notamment rectale.

En particulier, cet excipient peut être adapté pour l'obtention d'une composition sous forme d'une solution huileuse, d'une émulsion eau-dans-huile, une émulsion huile-dans-eau, une microémulsion, un gel huileux, un gel anhydre, une crème, une dispersion de vésicules, de microcapsules ou de microparticules, ou encore de gellules ou de capsules molles de gélatine ou végétales.

De préférence, on utilise un excipient adapté pour une administration par voie topique externe ou par voie rectale.

L'effet avantageux d'inhibition de l'activité de la 5α-réductase fourni par l'utilisation selon l'invention permet de destiner la composition ainsi préparée à des traitements thérapeutiques, notamment dermatologiques, et cosmétiques.

Ainsi, l'utilisation selon l'invention est caractérisée en ce que la composition est destinée au traitement des pathologies et/ou des désordres cutanés liés à une exagération congénitale ou acquise de l'activité de la 5α-réductase.

En particulier, l'utilisation selon l'invention est caractérisée en ce que la composition est destinée au traitement de l'hypertrophie prostatique.

En outre, l'utilisation selon l'invention est caractérisée en ce que la composition est destinée au traitement de l'adénome prostatique.

L'utilisation d'un excipient adapté pour une administration par voie rectale comme décrit ci-dessus peut être particulièrement envisagée pour ces traitements de l'hypertrophie et/ou de l'adénome prostatique.

L'utilisation selon l'invention est également caractérisée en ce que la composition est destinée au traitement de l'acné.

L'utilisation selon l'invention est également caractérisée en ce que la composition est destinée au traitement de l'hyperséborrhée.

Enfin, l'utilisation selon l'invention est également caractérisée en ce que la composition est destinée au traitement de l'alopécie.

L'utilisation selon l'invention est également caractérisée en ce que la composition est destinée au traitement de l'hirsutisme.

La présente invention a encore pour objet une méthode de traitement cosmétique de la peau grasse, caractérisée en ce qu'on applique sur la peau une composition cosmétique contenant au moins un produit d'huile végétale tel que décrit ci-dessus.

L'invention a par ailleurs pour objet une méthode de traitement cosmétique autre qu'une méthode de traitement thérapeutique de la chute des cheveux, caractérisée en ce qu'on applique sur le cuir chevelu une composition cosmétique contenant au moins un produit d'huile végétale tel que décrit ci-dessus.

Enfin, l'invention a également pour objet une méthode de traitement cosmétique autre qu'une méthode de traitement thérapeutique de l'excès de pilosité, caractérisée en ce qu'on applique sur les zones de la peau présentant des excès de pilosité une composition cosmétique contenant au moins un produit d'huile végétale tel que décrit ci-dessus.

En effet, à l'opposé des traitements médicaux hormonaux, ces deux dernières méthodes de traitement cosmétique permettent d'améliorer l'apparence en réduisant de manière visible les phénomènes disgracieux de chute de cheveux liés à l'alopécie et les phénomènes d'excès de pilosités liés à l'hirsutisme.

Selon un mode de mise en oeuvre préféré de ces méthodes de traitements cosmétiques, le produit d'huile végétale est présent dans la composition selon une proportion comprise entre environ 0,001 et environ 100 % en poids (possibilité d'utilisation sous forme pure, sans excipient), de préférence entre environ 0,01 et environ 70 % en poids, et plus particulièrement encore entre environ 0,1 et 10 % en poids, par rapport au poids total de la composition.

Avantageusement, la composition cosmétique appliquée selon la méthode cosmétique de l'invention contient en outre au moins un excipient cosmétiquement acceptable tel que décrit ci-dessus.

Enfin, l'invention a encore pour objet l'utilisation d'au moins un produit d'huile végétale tel que décrit ci-dessus, en tant qu'additif dans un aliment pour l'être humain et/ou l'animal. Cette utilisation alimentaire est de préférence caractérisée en ce que le produit d'huile végétale est présent dans l'aliment selon une proportion comprise entre environ 0,001 et environ 100 % en poids, de préférence entre environ 0,01 et environ 70 % en poids, et plus particulièrement encore entre environ 0,1 et 10 % en poids, par rapport au poids total de l'aliment.

Les exemples suivants sont destinés à illustrer la présente invention et ne doivent en aucun cas être interprétés comme pouvant en limiter la portée.

A moins qu'il n'en soit précisé autrement, les pourcentages indiqués dans les exemples suivants sont des pourcentages en poids.

### Exemple 1 : préparation de produits d'huile végétale

### 1.1) Oléodistillat (concentrat) d'huile de tournesol

On prépare un oléodistillat de tournesol par distillation moléculaire dans un distillateur moléculaire de type centrifuge d'une huile de tournesol alimentaire du commerce. Les conditions de distillation sont les suivantes :
- température : 220 °C;
- pression : 10⁻³ mmHg;
- taux de distillation : 6,7 % massique;
- débit d'alimentation : 18 kg/h.

Le distillat obtenu, l'oléodistillat de tournesol, présente une teneur en insapponifiable d'environ 6,2 % en poids, la partie restante étant composée par les triglycérides de l'huile de tournesol. L'Oléodistillat ainsi obtenu est dénommé "Oléodistillat de tournesol-1 ".

### 1.2) Oléodistillat (concentrat) d'huile d'avocat

On procède comme ci-dessus pour l'oléodistillat d'huile de tournesol, à partir d'une huile d'avocat, les conditions de distillation étant les suivantes :
- température 230 °C;
- pression : 10⁻³ mmHg;
- taux de distillation : 10 % massique;
- débit d'alimentation : 20 kg/h.

Le distillat obtenu, l'oléodistillat d'huile davocat, présente une teneur en insapponifiable d'environ 50 % en poids, la partie restante étant composée par les acides gras libres et les triglycérides de l'huile d'avocat.

Ce produit est dénommé ci-après "Oléodistillat d'avocat-1".

### 1.3) Oléodistillat (concentrat) d'huile de lupin

On procède comme ci-dessus pour l'oléodistillat d'huile de tournesol, à partir d'une huile de lupin, les conditions de distillation étant les suivantes :
- température : 270 °C;
- pression : 10⁻³ mmHg;
- taux de distillation : 2,7 % massique;
- débit d'alimentation : 18 kg/h.

Le distillat obtenu, l'oléodistillat d'huile de lupin, présente une teneur en insapponifiable d'environ 58 % en poids, la partie restante étant composée par les acides gras libres et les triglycérides de l'huile de lupin.

Ce produit est dénommé ci-après "Oléodistillat de lupin-1".

### 1.4) Insaponifiable d'avocat

100 g d'avocat coupé en lamelles de 5 mm d'épaisseur environ sont soumis aux opérations suivantes :

### 1.4.1) Traitement thermique du fruit

On place dans une étuve régulée à 80 °C les fruits découpés pendant 24 heures, puis on les broie.

### 1.4.2) Obtention de l'huile

La poudre obtenue à l'étape précédente est extraite à l'hexane par pression à froid dans une presse à vis de type « KOMET ». Le tourteau est éliminé et la solution hexanique est évaporée sous pression réduite. L'huile récupérée est filtrée sur «Büchner » puis stockée sous azote. On obtient ainsi 20 g d'huile d'avocat.

### 1.4.3) Concentration

On utilise un distillateur à film raclé tel que décrit ci-dessus, commercialisé par la société Leybold sous la dénomination "KDL 4". Il s'agit d'un appareil en verre, équipé d'une chambre de distillation dotée d'un racleur tournant, permettant l'étalement en continu sur la surface d'évaporation (surface chaude) du produit à traiter. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, ... ). La récupération des résidus et des distillats dans des ballons en verre, se fait par écoulement gravitationnel.

La température de distillation est d'environ 230 °C avec une pression de l'ordre de 10⁻³ mmHg. La vitesse de rotation de l'arbre est de 200 t/min et le débit d'alimentation est de 7 ml/mn.

### 1.4.4) Saponification

50 g de concentrat d'huile obtenu comme à l'étape précédente sont mélangés à 25 ml de potasse 12N, 100 ml d'éthanol et mis au reflux pendant 4 heures. On ajoute 175 ml d'eau à la phase hydroalcoolique, puis on ajoute 175 ml de dichloroéthane et on agite, puis on laisse décanter. On récupère ensuite la phase organique. Cette opération est répétée 5 à 6 fois. Les phases organiques sont réunies, lavées à l'eau et le solvant évaporé.

On obtient ainsi 20 g d'insaponifiable. Ce produit est dénommé ci-après "Insaponifiable d'avocat-1".

On peut bien entendu, pour une préparation à l'échelle industrielle, remplacer les étapes d'extraction en ampoule par une extraction en continu dans un apareil d'extraction liquide-liquide en continu tel que colonne pulsée, mélangeur décanteur ou équivalents.

### 1.5) Insaponifiable de soja

Dans un réacteur de type Grignard, 100 kg de concentrat ou oléodistillat d'huile de soja sont saponifiés à reflux en présence de 40 kg de potasse aqueuse (à 50%) et de 200 litres d'alcool éthylique. Après saponification, le mélange réactionnel est dilué par ajout de 300 kg d'eau déminéralisée puis envoyé dans une colonne d'extraction pulsée, à contre-courant. Le solvant mis en oeuvre est le dichloro-éthane (DCE) rapport solvant/solution hydro-alcoolique =1. Après extraction, la phase organique est lavée dans une colonne de lavage alimentée en continu par de l'eau déminéralisée. Le DCE, solvant d'extraction, est enfin séparé de l'insaponifiable brut dans un évaporateur de type flot tombant.

Après évaporation, l'insaponifiable subit une nouvelle étape de désolvation poussée ainsi qu'une étape de désodorisation à haute température.

L'insaponifiable ainsi obtenu est dénommé ci-après "Insaponifiable de soja-1".

### 1.6) Mélange d'insaponifiables d'avocat et de soja

On utilise le mélange d'insaponifiables d'huiles d'avocat et de soja tel que commercialisé par la société Laboratoires Pharmascience sous la dénomination "Piascledine 300®" qui consiste en un mélange de 33,3% en poids d'insaponifiable d'avocat et de 66,6% en poids d'insaponifiable de soja, par rapport au poids total du mélange (les 0,1% restants étant constitués de silice colloïdale et de butylhydroxytoluène).

Ce mélange est dénommé ci-après "Insaponifiables d'avocat et de soja-1".

### 1.7) Lipides furaniques d'avocat

On prépare un insaponifiable d'avocat comme décrit dans le brevet FR-2 678 632. Sa composition est la suivante :
- alcools gras polyhydroxylés 24,3 %
- lipides furaniques 55,5 %
- stérols 3,1 %
- squalène 1,4 %
- autres 15,7%(1)
(1) acides gras libres, hydrocarbures, tocophérols, cétones grasses et pigments lourds

On soumet cet insaponifiable à une distillation moléculaire à l'aide du distillateur moléculaire à film raclé commercialisé par la société Leybold sous la dénomination "KDL4". Les conditions de distillation sont les suivantes :
- température surface chaude: 108°C
- pression : 10⁻³ mm Hg
- vitesse de rotation de l'arbre: 240 t/min.
- débit d'insaponifiable d'avocat: 400 ml/h
Rendement en distillat: 48,6 %
Composition du distillat :
- alcools gras Polyhydroxylés : **n.m.**
- lipides furaniques 99,1 %
- stérols n.m.
- squalène n.m.
- autres 0,9 % **(1)**
(1) acides gras libres, hydrocarbures et cétones grasses
(**"n.m."** : non mesurable, c'est à dire une teneur inférieure à 0,05%)

Il s'agit donc d'un distillat très riche en lipides furaniques dans la mesure où la teneur de ces dernier excède 99 %. Ce distillat est dénommé par la suite "Lipides-1 ".

### Exemple 2 : Evaluation de l'activité inhibitrice sur l'activité de la 5α-réductase par mesure du taux de 5-dihydrotestostérone formée à partir de la testostérone par les cellules DU145.

### 1) Materiel et méthodes

### 1.1) Matériel

Les cellules prostatiques DU145 sont issues d'une lignée tumorale obtenue à partir d'un carcinome de la prostate (N° ATCC HTB 81). Le milieu MEM (réf.0410265), la glutamine et la gentamycine viennent de chez Gibco. Le sérum de veau foetal (SVF) vient de chez DAP et est utilisé décomplémenté (45 mm à 56°C). les plastiques servant à la culture (boîtes et plaques) viennent de chez Costar. La testostérone vient de chez Sigma.

### 1.2) Méthode

### 1.2.1) Préparation des gammes de produits

Une solution mère en éthanol à 10 mg/ml est préparée à partir de chacun des produits testés.

La gamme de concentration utilisée pour les expériences est la suivante : 0, 5, 10, 50, 100 et 500 microgrammes/ml. (Dilution effectuée dans le milieu de culture).

Le volume d'extrait ajouté par puits étant de 20 **microlitres**/puits, les solutions à préparer sont concentrées 50X.

### Préparation de la Testostérone

Une solution mère de testostérone à 10 mM est préparée dans l'éthanol. Au moment de son utilisation, cette solution est diluée au 1:1000 dans le milieu de culture et 10 microlitres sont ajoutés par puits.

### 1.2.2) Expérience d'inhibition de la 5α-réductase des DU 145

Les cellules prostatiques DU145 sont cultivées à 37°C, 5% CO₂ dans un milieu MEM contenant de la glutamine (2mM), de la gentamycine (50 microgrammes/ml) et 10% de SVF. Leur taux de sous-culture est de 1:10.

Avant de lancer l'expérimentation, les cellules sont mises en culture dans des plaques 6 puits à raison de 2.10⁵ DU145 par puits/1 ml de milieu ne contenant que 1% de SVF. Les cellules sont maintenues 3 jours à 37°C, 5% CO₂. Le jour de l'expérience, le milieu de culture contenu dans les puits est éliminé et remplacé par du milieu neuf contenant 1% de SVF. La testostérone (0,1 micromolaire final) ainsi que les extraits aux différentes concentrations sont ajoutés au milieu à raison de 10 et 20 microlitres/puits respectivement. (Les puits "contrôles" correspondent à des cellules incubées en présence de testostérone et d'un équivalent Ethanol. Ceci permet de soustraire l'effet du solvant sur les cultures et de déterminer le pourcentage de DHT formée en absence d'inhibiteur). Les cellules sont alors incubées à 37°C, 5% CO₂. Au bout de 3 heures, les sumageants de culture sont collectés et congelés à -80°C jusqu'au dosage.

### Mesure du taux de DHT formée

Principe: extraction des produits lipophiles par l'éther, concentration des échantillons en DHT par chromatographie d'affinité et dosage radio-immunologique

### Préparation des échantillons

- Après avoir agité au vortex les prélèvements, introduire les échantillons dans des flacons "SEPEX"
- Ajouter dans chaque tube 0,1 ml de la solution radioactive"3H-Rdt" (pour évaluation du rendement d'extraction). Boucher les flacons, les agiter un par un au vortex.
- Laisser reposer 30 min à température ambiante. Puis agiter de nouveau chaque flacon au vortex.
- Ajouter dans chaque flacon : 5 ml d'éther éthylique.
- Boucher les flacons et les agiter manuellement de façon énergique. Laisser décanter quelques minutes.
- Congeler les phases aqueuses à -30°C, pendant au moins 1 heure.
- Recueillir la phase éthérée dans un tube à essai en boro-silicate de 5 ml correspondant.
- Evaporer totalement la phase éthérée à l'aide du système évaporateur + bain-marie à 37°C.

### Séparation de la DHT

- Préparation des colonnes : Préparer les colonnes dans des pipettes de culture en verre de 5 ml avec 10 cm de chromatolithe A.
- Rinçage des colonnes : 3 ml d'iso-octane pur combitips (3 fois), en laissant couler par simple gravité.
- Elution des extraits éthérés secs
   - Chaque extrait sec est repris par 1 ml d'iso-octane pur, vortexer vigoureusement. Attendre 15 min à température ambiante. Réagiter au vortex.
   - Lorsque les 3 ml d'iso-octane (lavage des colonnes) sont élués, transvaser les extraits éthérés secs repris par l'iso-octane sur la colonne. Laisser éluer.
   - Rincer chaque tube "extrait sec" avec 1 ml d'iso-octane pur combitips, vortexer vigoureusement. Attendre 15 min à température ambiante. Réagiter au vortex et transvaser dans la colonne comme précédemment.
   - Laver par 4 ml d'iso-octane pur.
- Recueil de la DHT
   - Préparer le solvant d'élution (mélange à 6% iso-octane/acétate d'éthyl: 94/6 (v-v))
   - Eluer par 6 ml (pipette) de ce mélange.
   - Recueillir l'éluat DHT dans les tubes à essais en boro-silicate de 5 ml identifiés.
- Traitement de l'éluat DHT : Evaporer le solvant de l'éluat à l'aide du système évaporateur-bain-marie (37°C)

### Dosage RIA

- Protocole de distribution : Rependre les échantillons par 0,5 ml de tampon RC, le Blanc par 1 ml de tampon Rcet, les Contrôles par 0,5 ml de tampon RC. Placer à l'étuve à 37°C 15 min Agiter de nouveau les tubes au sortir de l'étuve (1 min).
   Dans des tubes à hémolyse en verre identifiés de 5 ml, mettre dans l'ordre:
   * **Tampon :** Activité Totale (AT): 0,7 ml de tampon RC, Activité Non Spécifique (N): 0,2 ml de tampon RC, Gamme : seul le point 0 de la gamme (noté B0) comporte 0, 1 ml de tampon RC,
   * **Solution étalon** (1000 à 7,8 pg/tube) : 0,1 ml de la solution étalon respective.
   * 0,1 ml **d'extrait sec** repris dans le tampon
      - Puis, distribuer **l'anti-sérum** : 0,1 ml dans tous les tubes sauf AT et N.
      - Puis, distribuer **la solution de dosage "3HD" :** 0,1 ml dans tous les tubes.
      - Vortexer et recouvrir d'un parafilm.
      - Incubation à 4°C, pendant 1h30 minimum (24 h. maximum).
- Préparation du charbon-dextran : Mettre la suspension de charbon-dextran dans un bécher, puis, dans un bain d'eau glacé à 4°C, pendant au moins 1h30.
- Rendement de purification en DHT
   - Dans 6 petites fioles à scintillation (3 par série) déposer : 0,4 ml de tampon RC + 0,1 ml de la solution "3H-Rdt" (flacon du premier jour au réfrigérateur). Blancs : mettre 0,5 ml d'extrait sec reconstitué pour le blanc. Echantillons et contrôles : mettre 0,25 ml de tampon RC + 0,25 ml d'extrait.
   - Ajouter 5 ml de liquide à scintillation dans toutes les fioles.

### Séparation de la DHT Iibre de celle liée à l'anticorps

- Mettre la suspension de charbon-dextran sous agitation magnétique, dans une bassine d'eau glacée.
- Ajouter 0,5 ml de charbon-dextran dans tous les tubes sauf AT en 2 min maximum.
- Vortexer, remettre les tubes dans l'eau glacée. Attendre 10 min exactement. Centrifuger à 4°C, 3400 rpm, pendant 11 min
- Pipeter 0,5 ml de chaque surnageant (y compris AT) dans une petite fiole de comptage
- Ajouter 5 ml de liquide scintillant. Agiter, laisser s'équilibrer 30 min à température ambiante.
- Mettre à compter 2 min avec le compteur Beta (Beckman, LS 6000 SE).

### 2) Résultats

### 2.1) Evaluation de la conversion de la testostérone en 5-dihydrotestostérone par les cellules DU 145 - Détermination des IC 50

**Tableau 2**

| **Produit testé** | **IC 50 (µg/ml)** |
|---|---|
| Oléodistillat d'avocat-1 | 205 |
| Oléodistillat de lupin-1 | 928 |
| Insaponifiable d'avocat-1 | 233 |
| Insaponifiable de soja-1 | 367 |
| Insaponifiables d'avocat et de soja-1 | 605 |
| Serenoa repens (1) | 10 |

| | |
|---|---|
| (1) Produit de référence pour l'inhibition de la 5α-réductase | |

### Exemple 3 : compositions sous forme d'émulsions huiles-dans-eau utilisables selon l'invention

Les compositions 1.1 à 1.3 ci après ont chacune été préparé de la manière suivante.

Les composants constituant la phase aqueuse (eau et glycérine) sont placées au bain-marie à 75 °C. Les composants de la phase grasse, à l'exception du SEPIGEL 305, du SILICONE SF 1202 et du produit d'huile végétale (respectivement préparés ci-après sous les dénominations "Oléodistillat de tournesol-1 ", "Insaponifiables-1 " et "Lipides-1 "), sont placés au bain-marie à 75 °C. Juste avant de réaliser l'émulsification, on ajoute à la phase grasse le SILICONE 1202 et le produit d'huile végétale respectif. On réalise ensuite l'émulsion sous turbine à vitesse lente par incorporation de la phase grasse dans la phase aqueuse. Lorsque la préparation atteint la température de 60 °C, on ajoute le SEPIGEL 305 sous turbine à grande vitesse. On laisse ensuite refroidir la composition au repos jusqu'à température ambiante, avant son utilisation dans les essais décrits ci-après.

| **Composition 1.1 (formule INCI)** | **% (en poids)** |
|---|---|
| **Phase aqueuse** | |
| Eau | 67,3 |
| Glycérine | 4 |

| **Phase grasse** | |
|---|---|
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |

| **Oléodistillat de tournesol-1** | 2 |
|---|---|
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

| **Composition 1.2 (noms INCI)** | **% (en poids)** |
|---|---|
| **Phase aqueuse** | |
| Eau | 67,3 |
| Glycérine | 4 |

| **Phase grasse** | |
|---|---|
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |

| **Insaponifiables d'avocat et de soja-1** | **2** |
|---|---|
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

| **Composition 1.3 (noms INCI)** | **% (en poids)** |
|---|---|
| **Phase aqueuse** | |
| Eau | 69 |
| Glycérine | 4 |

| **Phase grasse** | |
|---|---|
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Lipides-1** | 0,3 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

## Revendications

1. Utilisation d'au moins un produit d'huile végétale choisi dans le groupe constitué par les oléodistillats d'huile végétale, les insaponifiables d'huile végétale, les lipides furaniques d'huile végétale et les mélanges de ces derniers, pour la préparation d'une composition destinée au traitement d'une pathologie choisie pami l'hypertrophie prostatique, l'adénome prostatique, l'acné, l'hyperséborrhée, l'alopécie et l'hirsutisme.

2. Utilisation selon la revendication 1, **caractérisée en ce que** les insaponifiables et les oléodistillats d'huile végétale sont choisis dans le groupe constitué par les insaponifiables et oléodistillats riches en tocophérols et/ou en phytostérols.

3. Utilisation selon l'une quelconque des revendications précédentes, **caractérisée en ce que** les oléodistillats d'huile végétale sont choisis dans le groupe constitué par les oléodistillats d'huile d'avocat, d'huile de soja, d'huile de lupin, d'huile de tournesol et les mélanges de ces derniers.

4. Utilisation selon l'une quelconque des revendications précédentes, **caractérisée en ce que** les insaponifiables d'huile végétale sont des insaponifiables d'avocat, de soja ou des mélanges de ces derniers.

5. Utilisation selon l'une quelconque des revendications précédentes, **caractérisée en ce que** l'insaponifiable d'huile végétale est un mélange d'insaponifiable d'huile d'avocat et d'insaponifiable d'huile de soja, le rapport pondéral d'insaponifiable d'huile d'avocat à l'insaponifiable d'huile de soja étant compris entre environ 0,1 et environ 9.

6. Utilisation selon l'une quelconque des revendications précédentes, **caractérisée en ce que** les lipides furaniques d'huile végétale sont des lipides furaniques de l'avocat.

7. Utilisation selon l'une quelconque des revendications précédentes, **caractérisée en ce que** le produit d'huile végétale est utilisé selon une proportion comprise entre environ 0,001 et environ 100 % en poids, par rapport au poids total de la composition.

8. Utilisation selon l'une quelconque des revendications précédentes, **caractérisée en ce que** la composition préparée comprend un excipient pharmaceutiquement, dermatologiquement ou cosmétiquement acceptable.

9. Utilisation selon la revendication 8, **caractérisée en ce que** l'excipient est adapté pour une administration par voie topique externe ou par voie rectale.

10. Utilisation selon l'une quelconque des revendications précédente s, **caractérisée en ce que** la composition est destinée au traitement des pathologies et/ou des désordres cutanés liés à une exagération congénitale ou acquise de l'activité de la 5α-réductase.

11. Utilisation d'une composition cosmétique contenant au moins un produit d'huile végétale tel que défini à l'une quelconque des revendications 1 à 6, autre qu'une méthode de traitement thérapeutique pour la fabrication de compositions pour un traitement cosmétique de la peau grasse.

12. Utilisation d'une composition cosmétique contenant au moins un produit d'huile végétale tel que défini à l'une quelconque des revendications 1 à 6, autre qu'une méthode de traitement thérapeutique, pour la fabrication de compositions pour un traitement cosmétique de la chute de cheveux.

13. Utilisation d'une composition cosmétique contenant au moins un produit d'huile végétale tel que défini à l'une quelconque des revendications 1 à 6 autre qu'une méthode de traitement thérapeutique pour la fabrication de compositions pour un traitement cosmétique de l'excès de pilosité.

14. Utilisation selon l'une quelconque des revendications 11 à 13, **caractérisée en ce que** le produit d'huile végétale est présent dans la composition selon une proportion comprise entre environ 0,001 et environ 100 % en poids, par rapport au poids total de la composition.

15. Utilisation selon l'une quelconque des revendications 11 à 13, **caractérisée en ce que** la composition cosmétique contient en outre au moins un excipient cosmétiquement acceptable.

16. Utilisation d'au moins un produit d'huile végétale tel que défini à l'une quelconque des revendications 1 à 6 autre qu'une méthode de traitement thérapeutique, en tant qu'additif dans un aliment pour l'être humain et/ou l'animal.

17. Utilisation selon la revendication 16, **caractérisée en ce que** le produit d'huile végétale est présent dans l'aliment selon une proportion comprise entre environ 0,001 et environ 100 % en poids, par rapport au poids total de l'aliment.

## Claims

1. Use of at least one plant oil product chosen from the group consisting of oil distillates of a plant oil, unsaponifiable fractions of a plant oil, furan-containing lipids of a plant oil and mixtures thereof, to prepare a composition intended for treating a pathology selected from prostate hypertrophy, prostate adenoma, acne, hyperseborrhea, alopecia and hirsutism.

2. Use according to Claim 1, **characterized in that** the unsaponifiable fractions and the oil distillates of plant oils are chosen from the group consisting of unsaponifiable fractions and oil distillates that are rich in tocopherols and/or phytosterols.

3. Use according to either one of the preceding claims, **characterized in that** the oil distillates of a plant oil are chosen from the group consisting of the oil distillates of avocado oil, of soybean oil, of lupin oil and of sunflower oil, and mixtures thereof.

4. Use according to any one of the preceding claims, **characterized in that** the unsaponifiable fractions of a plant oil are unsaponifiable fractions of avocado or of soybean, or mixtures thereof.

5. Use according to any one of the preceding claims, **characterized in that** the unsaponifiable fraction of a plant oil is a mixture of unsaponifiable fraction of avocado oil and of unsaponifiable fraction of soybean oil, the weight ratio of unsaponifiable fraction of avocado oil to the unsaponifiable fraction of soybean oil being between about 0.1 and about 9.

6. Use according to any one of the preceding claims, **characterized in that** the furan-containing lipids of a plant oil are furan-containing lipids of avocado.

7. Use according to any one of the preceding claims, **characterized in that** the plant oil product is used in a proportion of between about 0.001% and about 100% by weight relative to the total weight of the composition.

8. Use according to any one of the preceding claims, **characterized in that** the composition prepared comprises a pharmaceutically, dermatologically or cosmetically acceptable excipient.

9. Use according to Claim 8, **characterized in that** the excipient is suitable for external topical administration or rectal administration.

10. Use according to any one of the preceding claims, **characterized in that** the composition is intended for treating skin pathologies and/or disorders associated with a congenital or acquired exaggeration of the activity of 5α-reductase.

11. Use of a cosmetic composition comprising at least one plant oil product as defined in any one of Claims 1 to 6, other than a therapeutic treatment method, for preparing compositions for cosmetic treatment of greasy skin.

12. Use of a cosmetic composition comprising at least one plant oil product as defined in any one of Claims 1 to 6, other than a therapeutic treatment method, for preparing compositions for cosmetic treatment of hair loss.

13. Use of a cosmetic composition comprising at least one plant oil product as defined in any one of Claims 1 to 6, other than a therapeutic treatment method, for preparing compositions for cosmetic treatment of excess pilosity.

14. Use according to any one of Claims 11 to 13, **characterized in that** the plant oil product is present in the composition in a proportion of between about 0.001% and about 100% by weight relative to the total weight of the composition.

15. Use according to any one of Claims 11 to 13, **characterized in that** the cosmetic composition also contains at least one cosmetically acceptable excipient.

16. Use of at least one plant oil product as defined in any one of Claims 1 to 6 other than a therapeutic treatment method, as an additive in human food and/or animal feed.

17. Use according to Claim 16, **characterized in that** the plant oil product is present in the food in a proportion of between about 0.001% and about 100% by weight relative to the total weight of the food.

## Patentansprüche

1. Verwendung von wenigstens einem Pflanzenölerzeugnis, ausgewählt aus der Gruppe, welche aus den Oleodestillaten von Pflanzenölen, den unverseifbaren Anteilen von Pflanzenölen, den furanischen Lipiden von Pflanzenölen und den Mischungen dieser Letzteren gebildet wird, für die Herstellung einer Zusammensetzung, welche für die Behandlung einer Pathologie, ausgewählt unter Prostatavergrößerung, Prostataadenom, Akne, Hyperseborrhoe, Alopezie und Hirsutismus, bestimmt ist.

2. Verwendung nach Anspruch 1, **dadurch gekennzeichnet, dass** die unverseifbaren Anteile und die Oleodestillate von Pflanzenölen aus der Gruppe, welche aus den unverseifbaren Anteilen und Oleodestillaten, welche reich an Tocopherolen und/oder an Phytosterolen sind, gebildet wird, ausgewählt werden.

3. Verwendung nach einem der vorangegangenen Ansprüche, **dadurch gekennzeichnet, dass** die Oleodestillate von Pflanzenölen aus der aus den Oleodestillaten von Avocadoöl, Sojaöl, Lupinenöl, Sonnenblumenöl und den Mischungen von diesen Letzteren gebildeten Gruppe ausgewählt werden.

4. Verwendung nach einem der vorangegangenen Ansprüche, **dadurch gekennzeichnet, dass** die unverseifbaren Anteile von Pflanzenölen die unverseifbaren Anteile von Avocado, Soja oder Mischungen von diesen Letzteren sind.

5. Verwendung nach einem der vorangegangenen Ansprüche, **dadurch gekennzeichnet, dass** der unverseifbare Anteil von Pflanzenölen eine Mischung des unverseifbaren Anteils von Avocadoöl und des unverseifbaren Anteils von Sojaöl ist, wobei das Gewichtsverhältnis des unverseifbaren Anteils von Avocadoöl zu dem unverseifbaren Anteil von Sojaöl zwischen ungefähr 0,1 und ungefähr 9 einschließlich liegt.

6. Verwendung nach einem der vorangegangenen Ansprüche, **dadurch gekennzeichnet, dass** die furanischen Lipide von Pflanzenölen furanische Lipide von Avocado sind.

7. Verwendung nach einem der vorangegangenen Ansprüche, **dadurch gekennzeichnet, dass** das Pflanzenölerzeugnis entsprechend einem Anteil zwischen ungefähr 0,001 und ungefähr 100 Gew.-% einschließlich bezogen auf das Gesamtgewicht der Zusammensetzung eingesetzt wird.

8. Verwendung nach einem der vorangegangenen Ansprüche, **dadurch gekennzeichnet, dass** die hergestellte Zusammensetzung eine aus pharmazeutischer, dermatologischer oder kosmetischer Sicht annehmbare Trägersubstanz umfasst.

9. Verwendung nach Anspruch 8, **dadurch gekennzeichnet, dass** die Trägersubstanz für eine Verabreichung auf topischem äußerlichem Wege oder auf rektalem Wege angepasst ist.

10. Verwendung nach einem der vorangegangenen Ansprüche, **dadurch gekennzeichnet, dass** die Zusammensetzung für die Behandlung der Hautpathologien und/oder -störungen bzw. -erkrankungen, welche mit einer angeborenen oder erworbenen übermäßigen Aktivität der 5α-Reduktase verbunden sind, bestimmt ist.

11. Verwendung einer kosmetischen Zusammensetzung, welche wenigstens ein Pflanzenölerzeugnis, wie in einem der Ansprüche 1 bis 6 definiert, enthält, außer einem Verfahren zur therapeutischen Behandlung, für die Herstellung von Zusammensetzungen für eine kosmetische Behandlung von fettiger Haut.

12. Verwendung einer kosmetischen Zusammensetzung, welche wenigstens ein Pflanzenölerzeugnis, wie in einem der Ansprüche 1 bis 6 definiert, enthält, außer einem Verfahren zur therapeutischen Behandlung, für die Herstellung von Zusammensetzungen für eine kosmetische Behandlung von Haarausfall.

13. Verwendung einer kosmetischen Zusammensetzung, welche wenigstens ein Pflanzenölerzeugnis, wie in einem der Ansprüche 1 bis 6 definiert, enthält, außer einem Verfahren zur therapeutischen Behandlung, für die Herstellung von Zusammensetzungen für eine kosmetische Behandlung von übermäßiger Behaarung.

14. Verwendung nach einem der Ansprüche 11 bis 13, **dadurch gekennzeichnet, dass** das Pflanzenölerzeugnis in der Zusammensetzung entsprechend einem Anteil zwischen ungefähr 0,001 und ungefähr 100 Gew.-% einschließlich bezogen auf das Gesamtgewicht der Zusammensetzung vorhanden ist.

15. Verwendung nach einem der Ansprüche 11 bis 13, **dadurch gekennzeichnet, dass** die kosmetische Zusammensetzung außerdem wenigstens eine aus kosmetischer Sicht annehmbare Trägersubstanz enthält.

16. Verwendung von wenigstens einem Pflanzenölerzeugnis, wie in einem der Ansprüche 1 bis 6 definiert, außer einem Verfahren zur therapeutischen Behandlung, als Zusatzstoff in einem Nahrungsmittel für Menschen und/oder Tiere.

17. Verwendung nach Anspruch 16, **dadurch gekennzeichnet, dass** das Pflanzenölerzeugnis in dem Nahrungsmittel entsprechend einem Anteil zwischen ungefähr 0,001 und ungefähr 100 Gew.-% einschließlich bezogen auf das Gesamtgewicht des Nahrungsmittels vorhanden ist.
